# EUROPÄISCHE PATENTSCHRIFT

(11) **EP 1 220 834 B1**
(45) Veröffentlichungstag und Bekanntmachung des Hinweises auf die Patenterteilung: **20.08.2003**
(21) Anmeldenummer: 00969507.3
(22) Anmeldetag: 16.10.2000
(51) Int. Cl.: C07C 253/00, C07C 255/19

(54) **VERFAHREN ZUR HERSTELLUNG VON CYANESSIGSÄUREESTERN**
METHOD FOR THE PRODUCTION OF CYANOACETIC ACID ESTERS
PROCEDE DE PREPARATION D'ESTERS DE L'ACIDE CYANOACETIQUE

(30) Priorität: 15.10.1999 EP 99120488; 13.10.2000 US 239921 P
(43) Veröffentlichungstag der Anmeldung: 10.07.2002
(73) Patentinhaber: Lonza AG, 4052 Basel (CH)
(72) Erfinder: CHEN, Peter, CH-8053 Zürich (CH); MÜLLER, André, CH-8954 Geroldswil (CH); GILBERT, Thomas, CH-8008 Zürich (CH)
(86) Internationale Anmeldenummer: EP0010141
(87) Internationale Veröffentlichungsnummer: WO01028983

(56) Entgegenhaltungen:
- EP-A- 0 032 078
- EP-A- 0 999 206
- US-A- 2 985 682

## Beschreibung

Die Erfindung betrifft ein Verfahren zur Herstellung von Cyanessigsäureestern der Formel

N≡C-CH₂-COOR (I),

worin R eine C₁₋₄-Alkylgruppe bedeutet.

Cyanessigsäureester sind wichtige Zwischenprodukte, zum Beispiel für die Synthese von α-Cyanacrylaten, Vitamin B₆, Guanin, Coffein, Allopurinol, Cyclobarbital, Folsäure, Theophyllin und Trimethoprim, um nur einige der bekanntesten Produkte zu nennen.

Die klassische Synthese von Cyanessigsäureestern geht von Chloressigsäure oder deren Estern aus, welche mit Cyanwasserstoff oder Cyaniden umgesetzt und gegebenenfalls anschliessend verestert werden. Ein Nachteil dieser Verfahren ist die Verwendung von Cyanwasserstoff oder Cyaniden, welche bekanntlich sehr toxisch sind.

Aufgabe der vorliegenden Erfindung war die Bereitstellung eines alternativen Verfahrens, das ohne Verwendung von Blausäure auskommt.

Erfindungsgemäss wird diese Aufgabe durch das Verfahren nach Patentanspruch 1 gelöst.

Es wurde überraschend gefunden, dass 3,3'-Bisisoxazol der Formel 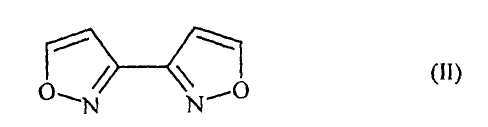 durch Pyrolyse in der Gasphase unter vermindertem Druck in Gegenwart von Alkoholen der Formel

R-OH (III),

worin R die oben genannte Bedeutung hat, direkt zu Cyanessigsäureestern (I) umgesetzt werden kann.

Alternativ kann die Pyrolyse auch in Abwesenheit von Alkohol durchgeführt und das erhaltene Pyrolysat mit dem Alkohol (III) umgesetzt werden.

Bei der nachträglichen Umsetzung mit dem Alkohol wird diese vorzugsweise unter Säurekatalyse, beispielsweise mit Schwefelsäure, durchgeführt.

3,3'-Bisisoxazol ist durch Umsetzung von Acetylen mit Stickoxiden leicht zugänglich (US-A-2 855 402).

Vorzugsweise wird das erfindungsgemässe Verfahren mit Methanol als Alkohol durchgeführt, so dass der Cyanessigsäuremethylester (R = Methyl) gebildet wird.

Die Pyrolysetemperatur beträgt vorzugsweise 500 bis 1000 °C, insbesondere 500 bis 700 °C, der Druck vorzugsweise 0,1 bis 100 Pa.

Die Pyrolyse kann beispielsweise in einem Quarzrohr durchgeführt werden, wobei dieses zur Verlängerung der Kontaktzeit und Erhöhung des Umsatzes vorteilhaft mit Füllkörpern wie beispielsweise Raschig-Ringen oder Einbauten versehen ist.

Die folgenden Beispiele verdeutlichen die Durchführung des erfindungsgemässen Verfahrens, ohne dass darin eine Einschränkung zu sehen ist.

### Beispiel 1

### Cyanessigsäuremethylester

In einem mit Raschig-Ringen aus Quarzglas gefüllten Quarzrohr (Rohr: *l*ₜₒₜₐₗ = 46 cm, *l*_{beheizt} = 30 cm, *d =* 2,4 cm; Raschigringe: *d*ₐ = 6 mm, *d*ᵢ = 3,5 mm, *l* = 10-15 mm) wurde eine Lösung von 485 mg (3,56 mmol) 3,3'-Bisisoxazol in Methanol (10 Gew.-%) bei 700 °C/<0,13 mbar (Pumpendruck ca. 0,001 mbar) pyrolysiert. Das Pyrolysat wurde in einer Kühlfalle bei 77 K kondensiert. Es enthielt neben 218 mg (45%) unumgesetztem 3,3'-Bisisoxazol 84 mg (24%) Cyanessigsäuremethylester. Bezogen auf die umgesetzte Menge an 3,3'-Bisisoxazol betrug die Ausbeute 43%.

### Beispiel 2

### Cyanessigsäureethylester

In der in Beispiel 1 beschriebenen Apparatur wurden 1,02 g (7,5 mmol) festes 3,3'-Bisisoxazol aus einem auf 60 °C geheizten Kolben während 90 min in das auf 500-700 °C geheizte Pyrolyserohr verdampft. Der in der Kühlfalle und dem kühlen Teil des Pyrolyserohrs abgeschiedene rotbraune Belag wurde in heissem Ethanol gelöst und die Lösung filtriert und zur Trockne eingeengt. Der Rückstand (297 mg) wurde in 20 ml Ethanol mit 0,7 ml konz. Schwefelsäure 5 h unter Feuchtigkeitsausschluss am Rückfluss gekocht und dann auf das halbe Volumen eingeengt. Anschliessend wurde die Lösung mit dem zehnfachen Volumen Wasser versetzt und mit Ethylacetat extrahiert. Nach dem Einengen wurden 183 mg dunkelbraunes Öl erhalten, das nach Chromatographie an Kieselgel 29 mg (3,3%) Cyanessigsäureethylester lieferte.

## Patentansprüche

1. Verfahren zur Herstellung von Cyanessigsäureestern der allgemeinen Formel
N≡C-CH₂-COOR (I),
worin R eine C₁₋₄-Alkylgruppe ist, **dadurch gekennzeichnet, dass** 3,3'-Bisisoxazol der Formel 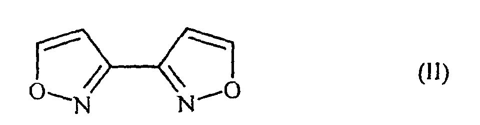 in Gegenwart eines Alkohols der allgemeinen Formel
R-OH (III),
worin R die oben genannte Bedeutung hat, in der Gasphase unter vermindertem Druck pyrolysiert wird.

2. Verfahren zur Herstellung von Cyanessigsäureestern der allgemeinen Formel
N≡C-CH₂-COOR (I),
worin R eine C₁₋₄-Alkylgruppe ist, **dadurch gekennzeichnet, dass** 3,3'-Bisisoxazol der Formel 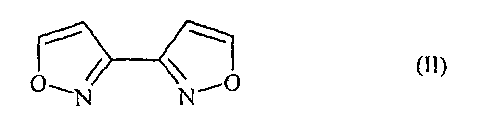 in der Gasphase unter vermindertem Druck pyrolysiert und das Pyrolysat mit einem Alkohol der allgemeinen Formel
R-OH (III),
worin R die oben genannte Bedeutung hat, umgesetzt wird.

3. Verfahren nach Anspruch 2, **dadurch gekennzeichnet, dass** die Umsetzung mit dem Alkohol (III) unter Säurekatalyse durchgeführt wird.

4. Verfahren nach einem der Ansprüche 1 bis 3, **dadurch gekennzeichnet, dass** R Methyl ist.

5. Verfahren nach einem der Ansprüche 1 bis 4, **dadurch gekennzeichnet, dass** die Pyrolyse bei einer Temperatur von 500 bis 700 °C und einem Druck von 0,1 bis 100 Pa durchgeführt wird.

## Claims

1. Process for the preparation of cyanoacetic acid esters of the general formula
N≡C-CH₂-COOR (I),
in which R is a C₁₋₄-alkyl group, **characterized in that** 3,3'-bisisoxazole of the formula 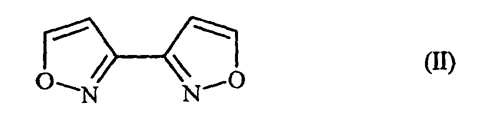 is pyrolysed in the gas phase under reduced pressure in the presence of an alcohol of the general formula
R-OH (III),
in which R has the abovementioned meaning.

2. Process for the preparation of cyanoacetic acid esters of the general formula
N≡C-CH₂-COOR (I),
in which R is a C₁₋₄-alkyl group, **characterized in that** 3,3'-bisisoxazole of the formula 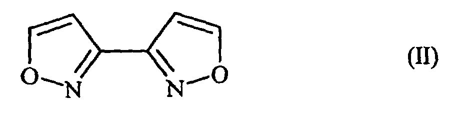 is pyrolysed in the gas phase under reduced pressure and the pyrolysate is reacted with an alcohol of the general formula
R-OH (III),
in which R has the abovementioned meaning.

3. Process according to Claim 2, **characterized in that** the reaction is carried out with the alcohol (III) under acid catalysis.

4. Process according to one of Claims 1 to 3, **characterized in that** R is methyl.

5. Process according to one of Claims 1 to 4, **characterized in that** the pyrolysis is carried out at a temperature of 500 to 700°C and a pressure of 0.1 to 100 Pa.

## Revendications

1. Procédé de préparation d'esters d'acide cyanoacétique de la formule générale
N≡C-CH₂-COOR (I),
dans laquelle R représente un radical alkyle en C₁ à C₄, **caractérisé en ce que** l'on pyrolyse du 3,3'-bisisoxazole de formule 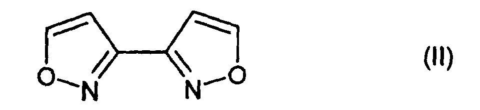 en présence d'un alcool de la formule générale
R-OH (III)
dans laquelle R a la signification ci-avant, en phase gazeuse et sous pression réduite.

2. Procédé de préparation d'esters d'acide cyanoacétique de la formule générale
N≡C-CH₂-COOR (I),
dans laquelle R représente un radical alkyle en C₁ à C₄, **caractérisé en ce que** l'on pyrolyse du 3,3'-bisisoxazole de formule 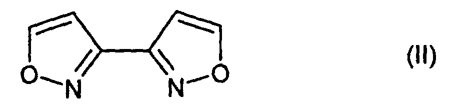 en phase gazeuse et sous pression réduite, et que l'on fait réagir le produit de pyrolyse avec un alcool de la formule générale
R-OH (III)
dans laquelle R a la signification ci-avant.

3. Procédé selon la revendication 2, **caractérisé en ce que** la réaction avec l'alcool (III) est entreprise avec catalyse acide.

4. Procédé selon l'une des revendications 1 à 3, **caractérisé en ce que** R est du méthyle.

5. Procédé selon l'une des revendications 1 à 4, **caractérisé en ce que** la pyrolyse est entreprise à une température de 500 à 700 °C et une pression de 0,1 à 100 Pa.
